# EUROPEAN PATENT APPLICATION

(11) **EP 4 032 465 A1**
(43) Date of publication of application: **27.07.2022**
(21) Application number: 21153357.5
(22) Date of filing: 25.01.2021
(51) Int. Cl.: A61B 5/00, A61B 5/024, A61B 5/03, A61B 5/08, A61B 5/11

(54) **A METHOD AND SYSTEM FOR MONITORING VITAL SIGNS OF AN INFANT**

(71) Applicant: Koninklijke Philips N.V., 5656 AG Eindhoven (NL)
(72) Inventor: LORATO, Ilde, 5656 AE Eindhoven (NL)
(74) Representative: Philips Intellectual Property & Standards

(57) **Abstract**

An infant monitoring system combines an infant soother having a sensor for detecting the location or sucking motion of the soother and a vital signs sensor for providing a vital signs sensor output. The vital signs sensor output is interpreted taking account of the sensing of the soother. In this way, it can be prevented that a soother sucking motion is incorrectly interpreted as a different vital signs measurement (such as heart rate or respiration rate) or otherwise corrupts the vital signs measurement.

## Description

### FIELD OF THE INVENTION

This invention relates to the monitoring of vital signs of an infant.

### BACKGROUND OF THE INVENTION

Monitoring infants' vital signs is necessary in hospital environments and it is becoming very common in home-care as well. Unobtrusive systems such as camera-based systems are being used in home environments, and research is being performed to allow the use of such systems also in Neonatal Intensive Care Units (NICUs) and Neonatal Medium Care Units (NMCU), thereby reducing the obtrusiveness of monitoring for infants.

Common unobtrusive solutions include various types of cameras (visible or thermal), radars, lasers, or pressure sensitive foils, and these can usually detect both respiration rate and heartbeat.

Camera solutions can be used to develop algorithms that evaluate heart and respiration rate by focusing on a specific part of the body. This may be difficult to implement as infants can be in many positions and are also often covered with blankets, which makes it more difficult to identify the skin or different body regions. Therefore, solutions that automatically detect the part of the image that contains respiration or heartbeat related information are being developed.

Algorithms that automatically detect the Region Of Interest (ROI), or solutions which monitor an entire area, assume that only the periodic signals of interest will be in a specific frequency band being monitored. However, infants often have a soother or pacifier which they move in a periodic way as well.

This motion is called Non-Nutritive Sucking (NNS) and frequencies from around 10 sucks per minute up to 150 sucks per minute have been reported. Considering that the normal respiration rate of an infant is within the range 30 to 90 breaths per minute, and the heartrate is within the range 90 to 180 beats per minute, it is evident that the presence of NNS can produce errors in the estimation of these two vital signs. For example, detecting the NNS as respiration could falsely indicate the baby is in tachypnoea, or detecting the NNS instead of the heart rate could falsely indicate the baby is in bradycardia.

Some types of vital signs sensor generate a single 1D signal from a monitored area. In these cases, it is particularly challenging to differentiate between the signal of interest (e.g. heart rate, respiration rate) and other semi-periodic phenomena (e.g. NNS).

Electrical current monitoring techniques such as chest impedance and ECG are not affected by NNS, but these require electrodes on the skin. It would be desirable if the same could be achieved with other forms of vital signs monitoring, including the unobtrusive monitoring solutions mentioned above.

### SUMMARY OF THE INVENTION

The invention is defined by the claims.

According to examples in accordance with an aspect of the invention, there is provided an infant monitoring system, comprising:
an infant soother system, comprising:
   a soother portion; and
   a soother sensor providing a soother sensor signal for identifying a position or movement of the soother portion;
a vital signs sensor for providing a vital signs sensor output; and
a processor, configured to:
   interpret the vital signs sensor output taking account of the soother sensor signal, to obtain a vital signs measurement.

The use of the soother sensor signal means a sucking motion of the soother is prevented from being interpreted as the vital signs measurement and/or it prevents contamination of the vital signs measurement. The influence of the sucking motion of the soother portion on the processing of the vital signs sensor output is reduced or eliminated.

This system makes use of an infant soother (which is intended to cover any device sucked by an infant other than for feeding, and is otherwise known as a pacifier or dummy). There is a sensor for detecting the location or movement of the soother. The sensed information about the soother enables the system to inhibit the processing of motion of the soother when interpreting the vital signs sensor output or to correct the vital signs sensor output. Thus, a possible source of error is removed from or reduced in the vital signs measurement. The vital signs measurement is preferably a non-contact measurement.

The soother sensor may be attached to the soother portion or it may be a remote sensor.

The soother sensor signal for example represents a sucking motion of the soother, and the processor is configured to:
derive a frequency of a sucking motion applied to the infant soother from the soother sensor signal; and
interpret the vital signs sensor output taking account of the frequency of the sucking motion.

The sensor in this case is able to detect motion. The motion may be detected directly as a motion signal or indirectly as a pressure or contact signal. The soother sensor signal then enables the frequency of the NNS motion to be extracted. In this way, frequency filtering can be used to prevent that the NNS signal is incorrectly interpreted as a different vital signs measurement.

The vital signs sensor may comprise one of:
an image sensor for monitoring reflections from the infant;
a thermal sensor for imaging emissions from the infant; and
a radar sensor for monitoring reflections from the infant.

These are all possible non contact sensing approaches, which can be used to derive vital signs information.

The vital signs sensor may instead comprise a pressure sensitive film for monitoring pressure variations. This film may be incorporated in a cot mattress for example.

The soother sensor may comprise one of:
an accelerometer; and
a pressure sensor.

Thus, motion may be sensed by an accelerometer, or a pressure may be sensed which detect a cyclic sucking pressure applied by the infant.

The processor is preferably configured to derive a breathing signal of the infant and/or a pulse signal of the infant.

The processor is for example configured to interpret the vital signs sensor output by filtering out contributions at the sucking motion frequency. Thus, these frequency components can be prevented from giving false vital signs readings.

In the case of a 1D sensor signal, a narrow bandwidth may be filtered so that the signal of interest remains detectable. In the case of a 2D signal, portions of the that 2D signal may be suppressed so that the signal of interest remains.

The processor may be configured to filter out the contributions by combining the vital signs sensor output and the soother sensor signal by using adaptive noise cancelation, with the soother sensor signal functioning as a noise reference signal.

This is one approach for filtering out the soothing sensor signal.

The vital signs sensor may be for obtaining a 1 dimensional signal. In such a case, erroneous detection due to an interfering NNS signal is particularly likely without the interpretation approach of the invention.

The invention also provides an infant soother for use in the system defined above, comprising:
a soother portion; and
a soother sensor for detecting sucking motion of the infant soother for use in interpreting a vital signs sensor output by taking account of the frequency of the sucking motion.

This aspect provides a soother with built in sensor for use in the overall system.

The invention also provides a method of monitoring vital signs of an infant, comprising:
receiving a soother sensor signal identifying a position or movement of an infant soother;
receiving a vital signs sensor output; and
interpreting the vital signs sensor output taking account of the soother sensor signal, to obtain a vital signs measurement.

The soother sensor signal may represent sucking motion of the infant soother, and the method comprises:
deriving a frequency of the represented sucking motion; and
interpreting the vital signs sensor output taking account of the frequency of the sucking motion.

The method may comprise receiving a vital signs sensor output comprising one of:
monitored reflections from the infant;
thermal emissions from the infant;
radar reflections from the infant; and
pressure variations induced by motions of the infant.

The method may comprise receiving a soother sensor signal comprising:
an accelerometer signal; or
a pressure sensor signal.

The vital signs sensor output is for example interpreted to derive a breathing signal or pulse signal of the infant. The vital signs sensor output is for example interpreted by filtering out contributions at the sucking motion frequency.

The invention also provides a computer program for implementing the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an infant monitoring system for monitoring vitals signs of an infant;
Figure 2 shows the processing steps applied to an infrared camera signal;
Figures 3 to 6 show images to represent processing of infrared camera signals for an example in which a soother is present but not moved, with conventional processing;
Figures 7 to 10 show images to represent processing of infrared camera signals for an example in which a soother is present and moved, with conventional processing;
Figures 11 to 14 show images to represent processing of infrared camera signals for an example in which a soother is present and moved, with processing in accordance with the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an infant monitoring system that combines an infant soother having a sensor for detecting the location or sucking motion of the soother and a vital signs sensor for providing a vital signs sensor output. The vital signs sensor output is interpreted taking account of the sensing of the soother. In this way, it can be prevented that a soother sucking motion is incorrectly interpreted as a different vital signs measurement (such as heart rate or respiration rate) or otherwise corrupts the vital signs measurement.

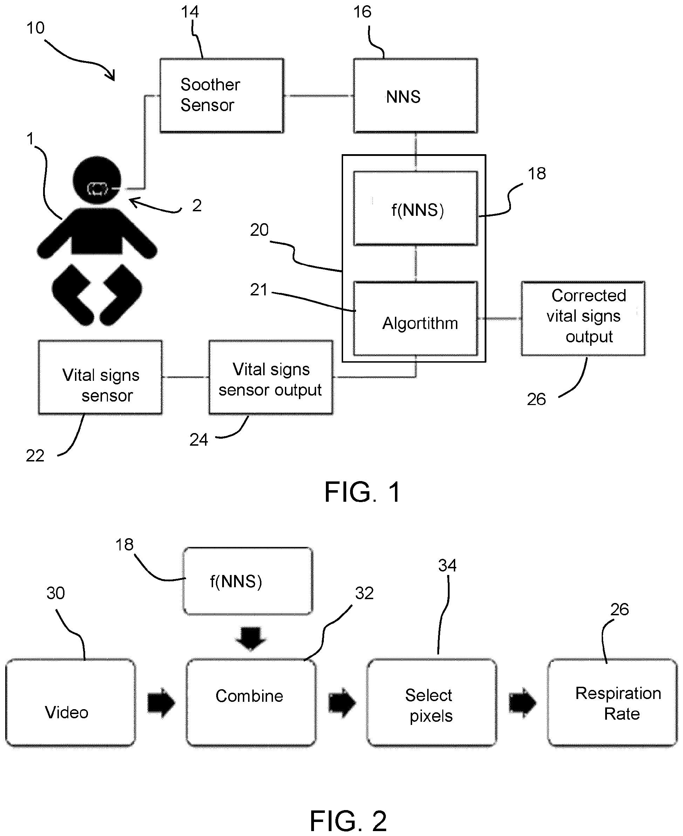

Figure 1 shows an infant monitoring system 10 for monitoring vitals signs of an infant 1.

The system comprises an infant soother 2 which has a soother sensor 14 (shown as a separate unit for clarity) for detecting either the location of the infant soother or the sucking motion of the infant soother. The infant soother has a soother portion, i.e. a teat on which the infant can suck and/or chew and a handle.

In a main set of examples, the soother sensor is a motion sensor mounted on or in the handle for detecting movement of the soother. In this case, the soother sensor is for registering (non-nutritive) sucking, NNS, of the infant and outputting a sucking signal. NNS is also in itself a vital signs of interest since it can indicate the presence of central nervous systems problems and can also be used to assess feeding readiness and oromotor development.

Many babies in Neonatal Medium Care Units are readmitted due to feeding problems. Thus, the monitoring of such signals could also be used to reduce infants' readmission or discharge when the infant is not ready. Indeed, it is known to monitor sucking behavior using a pressure sensor, and the sensed information can also be used to provide feeding training.

The soother sensor 14 is for example an accelerometer and/or a pressure sensor to capture the sucking motions of the infant.

The soother sensor 14 for example has a wireless transmitter for transmitting the sensor data to the remainder of the system for example using a short range wireless transmissions protocol such as Bluetooth.

The system also comprises a vital signs sensor 22 for providing a vital signs sensor output.

The vital signs sensor is for example for detecting physiological changes resulting from respiration and/or the heart beat of the infant. These physiological changes may be detected as movements, temperature variations due to flow, or skin color changes for example as can be detected by remote PPG solutions. Thus, the vital signs sensor is for detecting movement, temperature variations, or color variations resulting from respiration and/or the heart beat. The vital signs sensor is preferably an unobtrusive sensor, for example based on capturing reflections and/or emissions of at least part of the body of the infant. In one particular example, the vital signs sensor comprises 2D array of sensors, outputting sensor pixel data. In another example, the sensor may be a single sensor, outputting a 1D sensor signal.

In one example, the vital signs sensor 22 is an image sensor, for imaging reflections of the infant, for example at a wavelength or range of wavelengths in the band 400nm to 1000nm.

In another example, the vital signs sensor 22 is a thermal sensor, for imaging infrared emissions of the infant for example at wavelength or range of wavelengths in the band 2000 to 20000nm.

In another example, the vital signs sensor is a radar sensor for collecting reflections from the infant.

In yet another example, the vital signs sensor is a pressure sensitive film, for example integrated in a mattress, for collecting pressure variations.

In yet another example, the vital signs sensor is a remote temperature sensor for detecting temperature variations caused by airflow, such as respiratory flow.

In yet another example, the vital signs sensor is a remote PPG sensor (rPPG) for detecting skin color variations.

These are all examples of unobtrusive monitoring systems, which in themselves are known for collecting vital signs signals which result from physiological changes, such as the movement of parts of the body of a subject or temperature variations or skin color variations. Thus, these known systems will not be described in further detail. This is not intended to be an exhaustive list, and indeed the invention can be applied to any vital signs sensing modality for which the accuracy of the sensing may be compromised by a NNS motion.

By way of example, a raw breathing signal of the infant may be derived as indicated by I. Lorato et al., "Multi-camera infrared thermography for infant respiration monitoring," Biomed. Opt. Express, 2020. A raw pulse signal of the infant may be derived as indicated by W. Wang, S. Stuijk, and G. de Haan, "Living-Skin Classification via Remote-PPG," IEEE Trans. Biomed. Eng., vol. 9294, no. c, pp. 1-1, 2017.

In this example, based on motion sensing of the soother, the soother sensor 14 generates a non nutritive sucking signal 16, NNS, and a processor 20 derives a frequency, i.e. a dominant frequency 18, f(NNS), from the sucking signal.

The vital signs sensor generates a vital signs output 24.

The processor 20 operates an algorithm 21 for processing the vital signs sensor output 24 taking account of the frequency f(NNS) of the sucking motion. In this way, it can be prevented that the NNS signal is incorrectly interpreted as the vital signs measurement being separately monitored (such as heart rate or respiration rate). Thus, a possible source of error is removed from the vital signs measurement.

One particular implementation of the invention will now be described in more detail based on the use of an infrared camera to monitor the respiration rate of an infant, to show how the approach of the invention provides robustness for NNS related motion.

Figure 2 shows the processing steps applied to the infrared camera signal in more detail.

The vital signs sensor in this example generates a video stream 30 comprising multipixel images. The processor combines the raw vital signs sensor output and the sucking frequency signal 18 signal in step 32, and in step 34 makes a pixel selection that prevents the selection of pixels that show a dominant frequency similar to the sucking frequency f(NNS) computed from the sucking signal. The processor may for example use an adaptive noise cancellation approach, wherein the sucking signal is considered as the noise reference signal.

This operation in essence involves filtering out contributions at the sucking motion frequency. Thus, these frequency components can be prevented from giving false vital signs readings.

The vitals signs measurement of interest, in this example respiration rate, is then calculated from the modified, filtered, vital signs sensor output, using the known approaches as mentioned above.

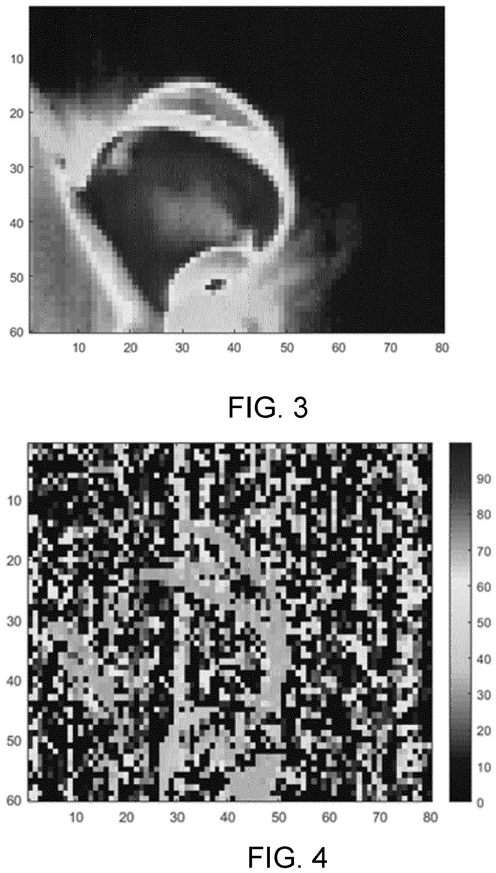

Figures 3 to 14 show how the system improves the robustness of deriving the desired vital signs measurement.

Figures 3 to 6 show an example in which the soother is present but not moved, with conventional processing.

Figure 3 shows a frame of a thermal imaging camera video of an infant with a soother. Figure 4 shows the main frequency components present in each pixel (color coded, hence showing different greyscale levels in the representation of Figure 4), obtained by performing a Fast Fourier Transform on the time domain signal of each pixel signal. The analysis clearly shows the presence of a dominant frequency component.

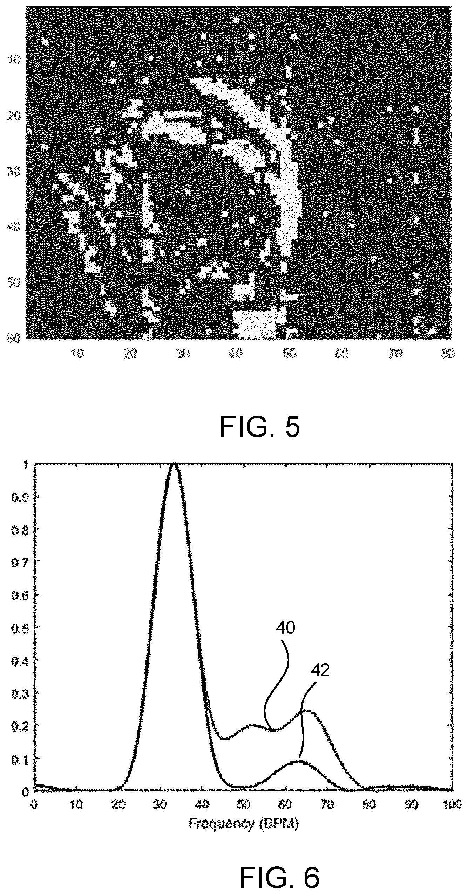

Figure 5 shows pixels that are automatically selected (using the algorithm presented in I. Lorato et al., "Multi-camera infrared thermography for infant respiration monitoring"). These pixels are used to extract the vital signs measurement.

Figure 6 shows the spectra of a reference signal 40 for measuring respiration based on bio-impedance measurement, and the signal obtained from the thermal imaging camera.

There is a close correspondence, with a respiration rate detected of around 35 breaths per minute.

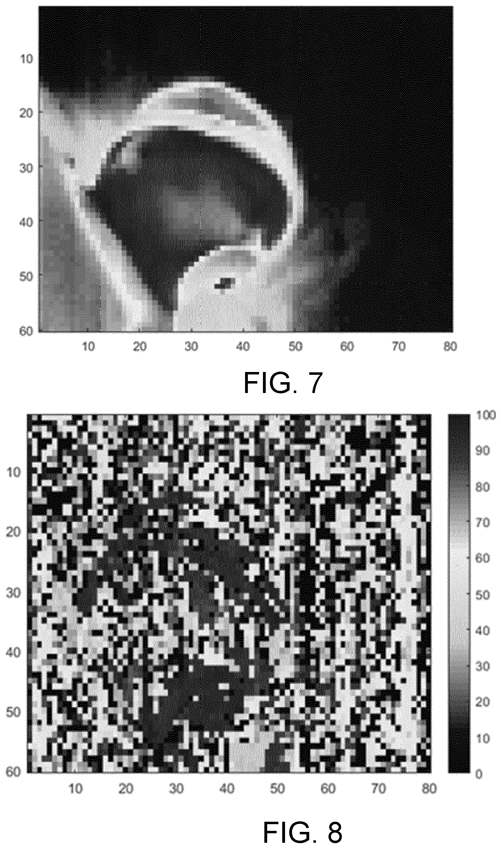

Figures 7 to 10 show the same images but for an example in which the soother is moved by a non-nutritive sucking motion. Again, conventional processing is used.

There are two main frequencies in the frequency analysis of Figure 8. There is one set of pixels containing a NNS signal at around 90 cycles per minute, in addition to a set of pixels containing respiration movement at around 35 breaths per minute. The image also contains respiratory flow information.

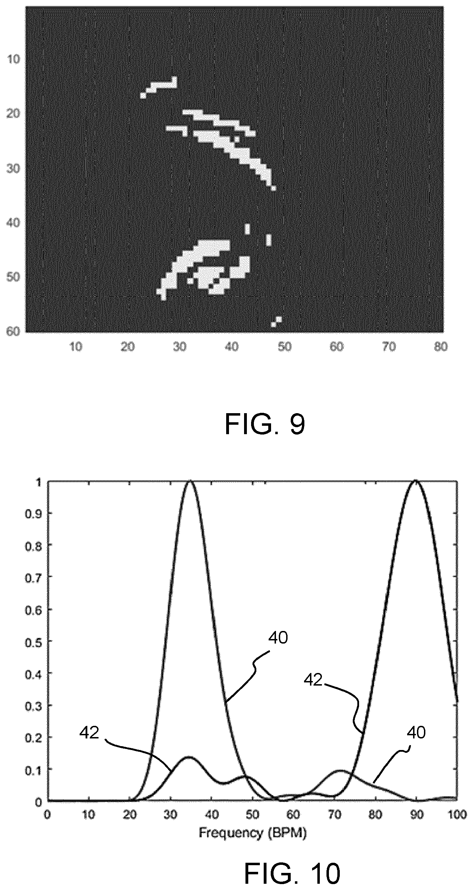

The standard processing algorithm then also selects pixels containing the NNS movement (in Figure 9), and the spectrum 42 from the thermal image processing as shown in Figure 10 includes the detection of an erroneous respiration rate caused by the presence of the NNS signal.

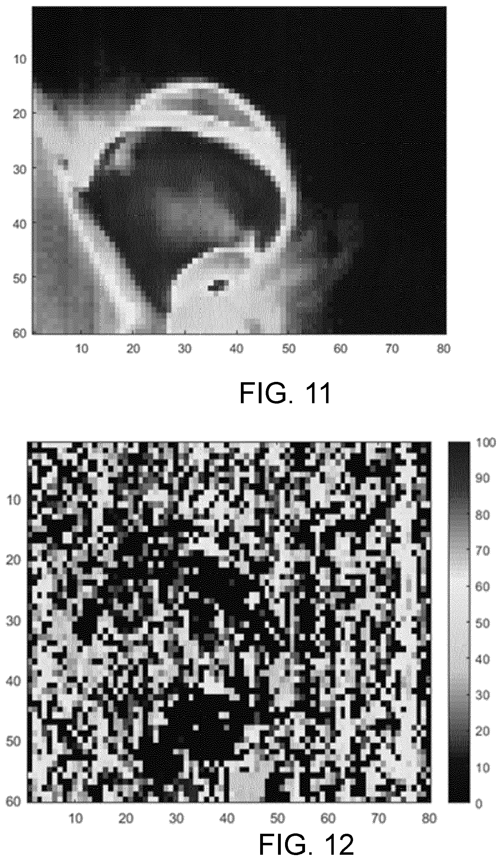

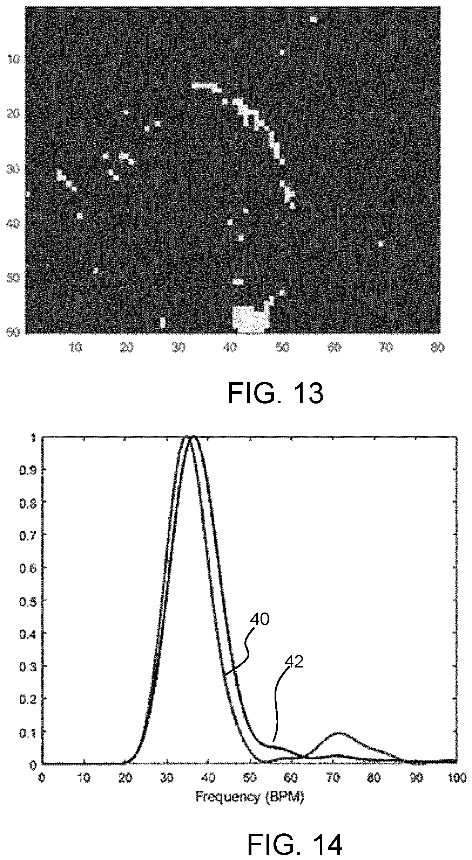

Figures 11 to 14 show the same images, again for an example in which the soother is moved by a non-nutritive sucking motion, but with the processing approach described above. These images show that by using the information on the NNS frequency from the soother before making the pixel selection choice, those frequencies from the selected pixels are suppressed from the image of Figure 12. As a result, a smaller set of pixels appears in Figure 13.

The desired robustness can be achieved simply by setting all of the pixels with the NNS frequency to zero in the plot of Figure 12. After removing the NNS pixels, the correct respiration rate is estimated as shown in Figure 14.

The example above uses a 2D sensor. Thus, the portions of the sensor signal which are to be filtered out can simply be removed, and the desired sensor signal of interest remains. The invention may however by implemented with a more simple vital signs sensor, for example outputting a 1D sensor signal.

In theory, the removal of the NNS signal components may suppress the signal of interest, if they happen to have the same frequency. However, such overlap will not last for a prolonged period as they are independent and non-correlated signals. If the vital signs measurement cannot be provided at a particular time, extrapolation or interpolation may be used to fill any gaps, or else the vital signs measurement may simply not be reported during those times.

Failure to report the vital signs measurement is not particularly alarming as the presence of the NNS signal itself indicates the wellbeing of the infant.

The examples explained above are based on a sensor forming part of the soother, and from which a frequency can be extracted. However, there are other ways to suppress the contribution of the soother to the vital signs sensor output and thereby achieve the same aim.

For example, the location of the soother may be identified so that in a 2D image sensor output (such as the infrared camera approach described above) the parts of the image corresponding to the soother may be ignored. Thus, pixels may be suppressed based on the knowledge that they correspond to sensor data collected from the soother, rather than using frequency analysis.

For example, a light source and near-infrared (NIR) camera may be used in combination with a highly reflective passive marker positioned on the soother to detect the NNS and distinguish it from the other periodic signals in the captured video of the vital signs sensor.

RGB cameras could also use an active marker positioned on the soother or a particular color or other feature of the soother could be used to identify it in captured videos.

The soother sensor may be implemented as part of the image processing of the vital signs sensor, for camera-based vital signs sensing solutions. Thus, the soother sensor may be part of the vital signs sensor algorithm. The soother may for example be identified by its shape. Assumption on the shape would be needed or else a calibration (training) would be necessary.

Thus, the soother sensor may be implemented as a remote sensor rather than a sensor fixed to the soother itself, and the soother sensor may even be implemented as part of the processing implemented by the vital signs sensor.

A detailed example has been given above for a thermal imaging camera solution. However, the approach of the invention, to suppress a signal contribution from a soother to a vital signs processing system, may be applied to any known unobtrusive/non-contact remote vital signs sensor system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An infant monitoring system (10), comprising:
an infant soother system, comprising:
a soother portion (2); and
a soother sensor (14) providing a soother sensor signal (16) for identifying a position or movement of the soother portion;
a vital signs sensor (22) for providing a vital signs sensor output (24); and
a processor (20), configured to:
interpret the vital signs sensor output (24) taking account of the soother sensor signal (16), to obtain a vital signs measurement.

2. The system of claim 1, wherein the soother sensor signal (16) represents a sucking motion of the soother portion, and the processor is configured to:
derive a frequency (18) of a sucking motion applied to the soother portion from the soother sensor signal; and
interpret the vital signs sensor output (24) taking account of the frequency of the sucking motion.

3. The system of claim 2, wherein the processor (20) is configured to interpret the vital signs sensor output (24) by filtering out contributions at the sucking motion frequency.

4. The system of claim 3, wherein the processor (20) is configured to filter out the contributions by combining the vital signs sensor output (24) and the soother sensor signal (6) by using adaptive noise cancelation, with the soother sensor signal functioning as a noise reference signal.

5. The system of any one of claims 1 to 4 wherein the vital signs sensor (22) comprises one of:
an image sensor for monitoring reflections from the infant;
a thermal sensor for imaging emissions from the infant; and
a radar sensor for monitoring reflections from the infant.

6. The system of any one of claims 1 to 5, wherein the vital signs sensor (22) comprises a pressure sensitive film for monitoring pressure variations.

7. The system of any one of claims 1 to 6, wherein the soother sensor (14) comprises one of:
an accelerometer; and
a pressure sensor.

8. The system of any one of claims 1 to 7, wherein the processor (20) is configured to derive a breathing signal of the infant or a pulse signal of the infant.

9. An infant soother for use in the system of any one of claims 1 to 8, comprising:
a soother portion; and
a soother sensor (14) for detecting sucking motion of the infant soother for use in interpreting a vital signs sensor output by taking account of the frequency of the sucking motion.

10. A method of monitoring vital signs of an infant, comprising:
receiving a soother sensor signal identifying a position or movement of an infant soother;
(30) receiving a vital signs sensor output; and
(32.34.26) interpreting the vital signs sensor output taking account of the soother sensor signal, to obtain a vital signs measurement.

11. The method of claim 10, wherein the soother sensor signal represents sucking motion of the infant soother, and the method comprises:
deriving a frequency of the represented sucking motion; and
interpreting the vital signs sensor output taking account of the frequency of the sucking motion.

12. The method of claim 10 or 11, comprising receiving a vital signs sensor output comprising one of:
monitored reflections from the infant;
thermal emissions from the infant;
radar reflections from the infant; and
pressure variations induced by motions of the infant.

13. The method of any one of claims 10 to 12, comprising receiving a soother sensor signal comprising:
an accelerometer signal; or
a pressure sensor signal.

14. The method of any one of claims 10 to 13, comprising interpreting the vital signs sensor output to derive a breathing signal or pulse signal of the infant.

15. A computer program comprising computer program code means which is adapted, when said program is run on a computer, to implement the method of any one of claims 10 to 14.
